# EUROPEAN PATENT SPECIFICATION

(11) **EP 3 865 460 B1**
(45) Date of publication and mention of the grant of the patent: **15.11.2023**
(21) Application number: 20198290.7
(22) Date of filing: 25.09.2020
(51) Int. Cl.: C02F 3/34, C12N 1/14, C12R 1/645, C02F 1/28, C02F 101/32, C02F 103/08

(54) **METHOD FOR PETROLEUM HYDROCARBON BIOREMEDIATION USING FUNGI**
VERFAHREN ZUR BIOLOGISCHEN SANIERUNG VON ERDÖLKOHLENWASSERSTOFF UNTER VERWENDUNG VON PILZEN
PROCÉDÉ DE BIOREMÉDIATION D'HYDROCARBURES DE PÉTROLE UTILISANT DES CHAMPIGNONS

(30) Priority: 03.06.2020 LT 2020527
(43) Date of publication of application: 18.08.2021
(73) Proprietor: Klaipeda University, 92294 Klaipeda (LT)
(72) Inventor: Katarzyte, Marija, 92359 Aukstkiemiai (LT); Paulauskiene, Tatjana, 91136 Klaipeda (LT)
(74) Representative: Klimaitiene, Otilija

(56) References cited:
- EP-A1- 1 619 173
- EP-A2- 1 464 626
- US-A- 4 415 661
- BARNES NATASHA MARIA ET AL: "Bioremediation potential of hydrocarbon-utilizing fungi from select marine niches of India", 3 BIOTECH, [Online] vol. 8, no. 1, 18 December 2017 (2017-12-18), XP055779914, DE ISSN: 2190-572X, DOI: 10.1007/s13205-017-1043-8 Retrieved from the Internet: URL:http://link.springer.com/content/pdf/1 0.1007/s13205-017-1043-8.pdf> [retrieved on 2021-02-25]

## Description

### TECHNICAL FIELD

The present invention relates to a method for petroleum hydrocarbon degradation in water using fungi strains isolated from sand and water of Baltic Sea coast samples.

### BACKGROUND ART

Increased number of shipments of crude oil in the Baltic and other seas results in an increase of their transhipments at oil terminals as well as an increasing number of incidents when crude oil, its products, alternative fuels and their blends are spilled into the environment. Petroleum hydrocarbons (PHs) are a major source of seawater contamination, which is harmful for marine ecosystems and human health.

The spread of spilled petroleum hydrocarbons is affected by hydrometeorological parameters (temperature, wind speed, wave parameters, water currents etc.) and the type of hydrocarbons as well as evaporation of the volatile fractions such as low molecular weight alkanes and monoaromatic hydrocarbons (McGenity et al., 2012). Spilled hydrocarbons form a film which is broken into droplets and dispersed through the water column, enhancing the biodegradation of hydrocarbons and dissolution of water-soluble fractions of petroleum. Turbulent seas cause water droplets to be suspended in the petroleum, resulting in water-in-oil emulsions, alternatively known as chocolate mousse, which is difficult to degrade because of its high viscosity and reduced surface area.

Photo-oxidation is another petroleum weathering process by which petroleum hydrocarbons, especially PAHs, react with oxygen in the presence of sunlight, resulting in structural changes that can, on one hand, lead to increased water solubility or, conversely, increase recalcitrance to biodegradation.

Sedimentation will generally only occur when oil adsorbs to particles owing to nearly all crude oils having a lower density than seawater.

However, natural biodegradation is one of the most important means by which oil is removed from the marine environment, especially the non-volatile components of crude oil and its products.

The main goal of using microorganisms in bioremediation is to reduce or eliminate toxic substances from contaminated sites through different pathways such as degradation, assimilation, or transpiration in the atmosphere, yielding nontoxic final products such as inorganic molecules, water, carbon dioxide, and microbial biomass. Due to their biological origin, they present some advantages compared to chemical surfactants, like better biocompatibility and biodegradability, reduced toxicity, activity and stability at extreme conditions of temperature, pH, and salinity.

The major benefit from using bioremediation processes is its contribution to the environment. It is an eco-friendly and sustainable process with almost no harmful side effects, the process does not involve transferring of contaminants to other environmental mediums. Moreover, the area of treatment can be larger than with other remedial technologies, because the treatment moves with the plume and can reach areas that would otherwise be inaccessible. As an *in situ* (versus *ex situ*) method, there is little secondary waste generated, reduced potential for cross-media transfer of contaminants and risk of human exposure to contaminated media. Furthermore, *in situ* bioremediation often costs less than other remedial technologies, it requires minimal equipment and less energy.

Microbial degradation methods show potential because microorganisms possess enzyme systems to degrade and use various PHs as a sole carbon and energy sources.

The biodegradation activity of microbes can be affected by the following environmental factors: temperature, oxygen, pH and nutrients. For successful biodegradation, microorganisms must develop a catabolic activity by the following processes: new metabolic capabilities developed by changes in genetics, or induction of specific enzymes and eclectic enrichment of microorganisms that are capable of converting the pollutants. When conditions are favourable to the microorganisms, biodegradation reaches a maximum level.

Nutrients are important components for a successful contaminant biodegradation, those include nitrogen, and phosphorus. Carbon comes from an organic source (PHs); hydrogen and oxygen are supplied from the water (Kalantary et al., 2014). In marine, brackish and freshwater environments, oil spills cause an increase of carbon levels and a decrease in nitrogen and phosphorus levels which can affect the biodegradation process. In brackish environments, nitrogen and phosphorus levels are low, thus, addition of nutrients is necessary to promote the biodegradation of contaminants (Atlas, 1995, Head & Swannell, 1999). In the case of marine environments, the addition of biostimulants is critical, as firstly they should target the microbes near the oil droplets in the seawater and secondly, they should not readily get diluted and washed out by the wave action.

The highest rates of petroleum hydrocarbon degradation in marine and freshwater environments is achieved at 20-30 °C and 15-20 °C, respectively.

Microorganisms, such as bacteria, fungi, and yeasts, whether from marine or soil environments, are capable of degrading diverse hydrocarbon compounds and are used in bioremediation processes.

Prior studies have examined the biodegradation of petroleum hydrocarbons by marine fungi by quantifying changes in the total mass of oil over time or by examining changes in the quantity of oil-derived compounds including straight chain n-alkanes, branched alkanes (e.g. pristane and phytane) as well as polycyclic aromatic hydrocarbons. Fungi are capable of degrading this structurally diverse range of crude oil-derived compounds by employing a variety of mechanisms including intracellular and extracellular enzymes.

Hydrocarbons that leak in water tend to spread and form a spot due to the wind and wave action; water in oil or oil in water ("foam") may form emulsions (Colwell et al., 1977). An important process in the absorption of PHs by microorganisms is the formation of an emulsion by their own production or by the help of biosurfactants.

Bacteria and fungi have been described as biosurfactant producers. Due to their biological origin, they present some advantages compared to chemical surfactants, such as better biocompatibility and biodegradability, reduced toxicity, activity and stability at extreme conditions of temperature, pH, and salinity. Biosurfactants based on their constituent structures can be glycolipids, phospholipids, polysaccharides and lipid complexes, lipoproteins, and cross-linked and hydroxylated fatty acids and may be whole cellular structures (Effendi et al., 2018).

A wide variety of fungi are known to be important in initiating biodegradation of HMW PAHs in terrestrial environments by co-metabolism using a battery of enzymes (McGenity et al., 2012). PHs degradation by fungi in the terrestrial environment is well established, however the diversity of species and the associated mechanisms and degradation pathways are yet to be fully explored. Estimates of fungal diversity range from 1.5 to 3.5-5.1 million species. Although fungi are considered to be largely terrestrial, they have been found in aquatic environments (freshwater and marine), but in general have received little attention despite a potentially major role in coastal PHs degradation (McGenity et al., 2012). Owing to the ubiquity and persistence of PHs in the coastal marine environment, the degradation of petroleum hydrocarbons by marine fungi is of particular interest.

Fungi have different degradation mechanisms than bacteria. In the fungi systems, the degradative enzyme is secreted by the fungus from its mycelia and is called an extracellular enzyme. Thus, the biodegradation process takes place outside the fungal cell. With these mechanisms they can overcome the problem of molecular size of pollutant compounds and toxicity of pollutant compounds against degrading microorganisms. Fungi are important in the initial phase of PHs degradation. Fungal enzymes like lignin peroxidases, manganese peroxidases, laccases and epoxide hydrolases, that probably evolved to breakdown other compounds such as lignin, fortuitously degrade PHs (McGenity et al., 2012). The effectiveness of the enzymes, based on their strong oxidative activity and low substrate specificity, depends on fungal species/strain, oxidative mechanism, lignocellulose nature and cultivation conditions (Galic et al., 2018).

Bioaugmentation, i.e., addition of microorganisms to the contaminated sites, is often considered a more practical method compared to other methods. The efficiency of bioaugmentation is dependent on inoculation of the appropriate amount of microbial biomass, the delivery method, and potential sorption to the surrounding media. One of the more promising techniques in this field is to use different carrier or support materials for cell immobilization to maintain sufficient microbial activity for a long period of time, thereby enabling higher bioremediation efficiency (Tyagi et al., 2011). Fungus belonging to *Aspergillus, Penicillium, Alternaria, Fusarium* genus are among the microorganisms with the ability to degrade petroleum hydrocarbons in aqueous environment (Ameen et al., 2016, Al-Hawash et al., 2019, Chulalaksananukul et al., 2006, Al-Hawash et al., 2018). However, to our knowledge until now the filamentous fungi isolates and their capability of biodegradation in aqueous environments were tested only in laboratory conditions, using small amounts of volumes, and no further application, for use in *ex situ* conditions, were developed. The main aspects for use of fungi isolates capable of degrading petroleum hydrocarbons in aquatic environments is that the method is cost-effective and environmentally friendly. Moreover, the production of fungal biomass, immobilisation, and addition of biostimulants, that target the fungi near the oil droplets in the water are not readily diluted and washed out by the wave action, the fungal biomass can be preserved until deployment time and they can be used in *ex situ* environment (bioaugmentation). US4415661A discloses a method for the biodegradation of petroleum materials using a fungus species. EP1464626A2 discloses a method for the reduction of organic matter from industrial effluents using filamentous fungi species. Barnes et al. ("Bioremediation potential of hydrocarbon-utilizing fungi from select marine niches of India", 3 BIOTECH, vol. 8, no. 1, 18 December 2017, DOI: 10.1007/s13205-017-1043-8) disclose the isolation of fungal species with an ability to degrade crude oil from marine substrates.

### BRIEF DESCRIPTION OF THE INVENTION

This invention describes the use of fungal biomass selected from the coastal areas of the Baltic sea for deployment in marine environment affected by a petroleum hydrocarbon spillage.

According to the method of using fungi derived from the coastal environment of the present invention, the decrease of the petroleum hydrocarbons in the water can be achieved. As an example, the present invention can be applied to the crude oil, alternative fuels and their blends clean-up. Furthermore, according to the present invention, it is also possible to use this method for additional application - for restoring the natural microflora at the point of application.

The method comprises selection and cultivation of specific species of fungi, evaluation of the severity and chemical composition of the spillage and timely use of the composition in the affected area.

The invention provides a method as defined in claim 1 for petroleum hydrocarbon degradation in water using fungi, where petroleum hydrocarbons to be degraded are brought into contact with an inoculum formed from at least the following components:
fungi strains isolated from sand and water of Baltic Sea coast samples, wherein the fungi strains are selected from *Clonostachys rosea, Alternaria rosae* or *Emericellopsis maritima;*
an environmentally friendly support material that acts as a solid substrate for the fungi and is a natural sorbent of hydrocarbons, wherein the support material on which at least one fungi strain is immobilized, is selected from: wheat straw, oat straw, rice straw, corn straw, barley straw, cereal straw wheat bran or sawdust;
and wherein *Emericellopsis sp.* is applied additionally for microflora restoration.

### BRIEF DESCRIPTION OF THE DRAWINGS

Features of the invention believed to be novel and inventive are set forth with particularity in the appended claims. The invention itself, however, may be best understood by reference to the following detailed description of the invention, which describes exemplary embodiments, given in non-restrictive examples, of the invention, taken in conjunction with the accompanying drawings, in which:

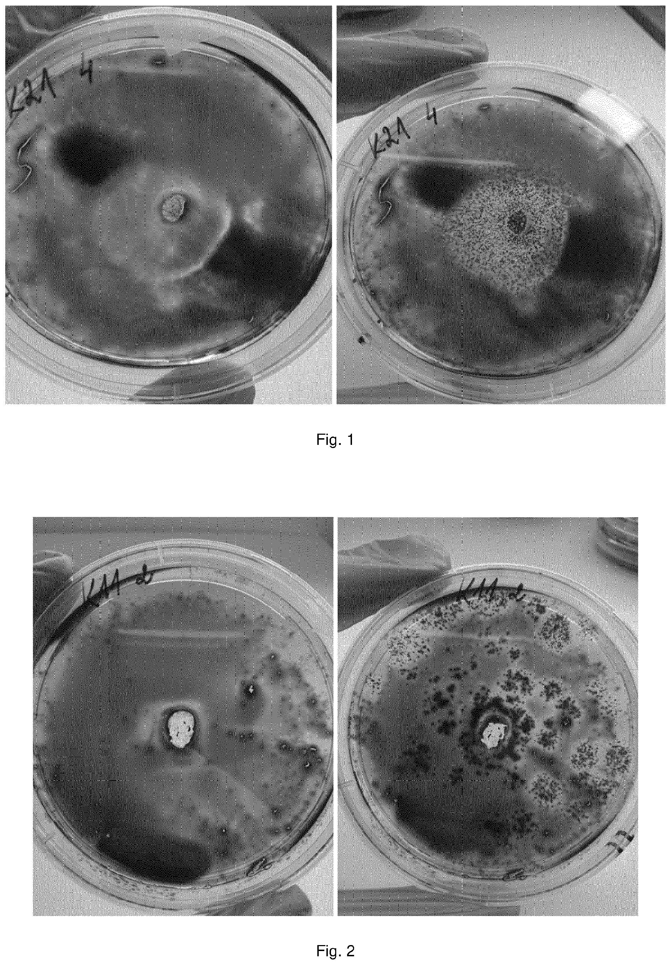

Fig. 1 shows *Alternaria rosea strain on water agar experiment day T2 and day T8.*
Fig. 2 shows *Clonostachys rosea* strain on water agar experiment day T4 and day T15.

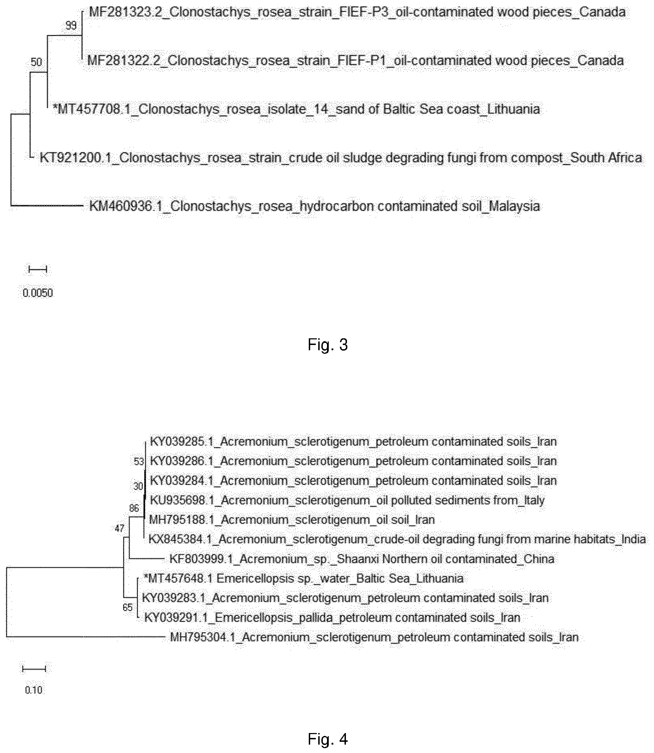

Fig. 3 shows *Clonostachys rosea* strain (*) sequence similarity with sequences of fungi derived from the petroleum hydrocarbon contaminated environment or used for its degradation, from the BLAST database. Neighbour-Joining method. The percentage of replicate trees in which the associated taxa clustered together in the bootstrap test (1000 replicates) are shown next to the branches. The tree is drawn to scale, with branch lengths in the same units as those of the evolutionary distances used to infer the phylogenetic tree. The evolutionary distances were computed using the Maximum Composite Likelihood method and are in the units of the number of base substitutions per site. Codon positions included were 1st+2nd+3rd+Noncoding. All ambiguous positions were removed for each sequence pair (pairwise deletion option). Evolutionary analyses were conducted in MEGA X [4].
Fig. 4 shows *Emercillopsis* sp. strain (*) sequence similarity with sequences of fungi derived from the petroleum hydrocarbon contaminated environment or used for its degradation, from the BLAST database. Neighbour-Joining method. The percentage of replicate trees in which the associated taxa clustered together in the bootstrap test (1000 replicates) are shown next to the branches. The tree is drawn to scale, with branch lengths in the same units as those of the evolutionary distances used to infer the phylogenetic tree. The evolutionary distances were computed using the Maximum Composite Likelihood method and are in the units of the number of base substitutions per site. Codon positions included were 1st+2nd+3rd+Noncoding. All ambiguous positions were removed for each sequence pair (pairwise deletion option). Evolutionary analyses were conducted in MEGA X [4].

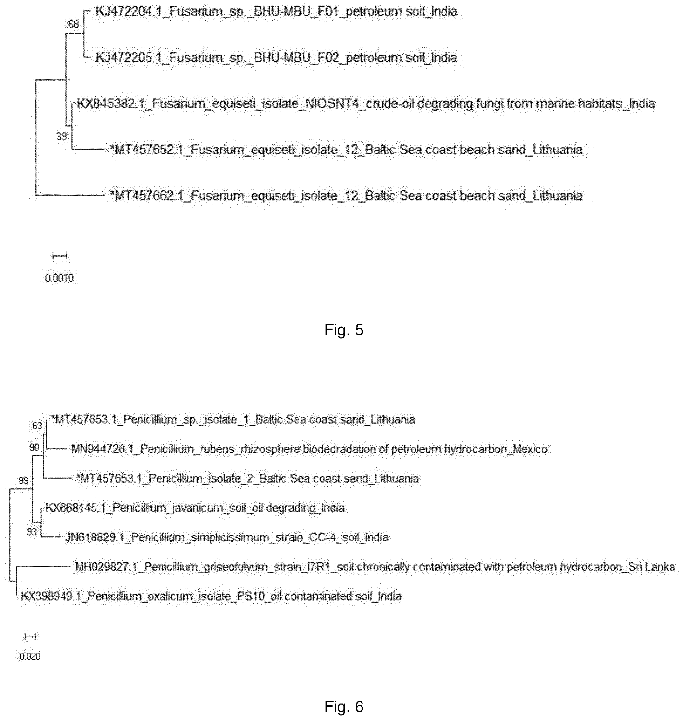

Fig. 5 shows *Fusarium equiseti* strains (*) sequence similarity with sequences of fungi derived from the petroleum hydrocarbon contaminated environment or used for its degradation, from the BLAST database. Neighbour-Joining method. The percentage of replicate trees in which the associated taxa clustered together in the bootstrap test (1000 replicates) are shown next to the branches. The tree is drawn to scale, with branch lengths in the same units as those of the evolutionary distances used to infer the phylogenetic tree. The evolutionary distances were computed using the Maximum Composite Likelihood method and are in the units of the number of base substitutions per site. Codon positions included were 1st+2nd+3rd+Noncoding. All ambiguous positions were removed for each sequence pair (pairwise deletion option). Evolutionary analyses were conducted in MEGA X [4].
Fig. 6 shows *Penicillium* sp. strains (*) sequence similarity with sequences of fungi derived from the petroleum hydrocarbon contaminated environment or used for its degradation, from the BLAST database. Neighbour-Joining method. The percentage of replicate trees in which the associated taxa clustered together in the bootstrap test (1000 replicates) are shown next to the branches. The tree is drawn to scale, with branch lengths in the same units as those of the evolutionary distances used to infer the phylogenetic tree. The evolutionary distances were computed using the Maximum Composite Likelihood method and are in the units of the number of base substitutions per site. Codon positions included were 1st+2nd+3rd+Noncoding. All ambiguous positions were removed for each sequence pair (pairwise deletion option). Evolutionary analyses were conducted in MEGA X [4].

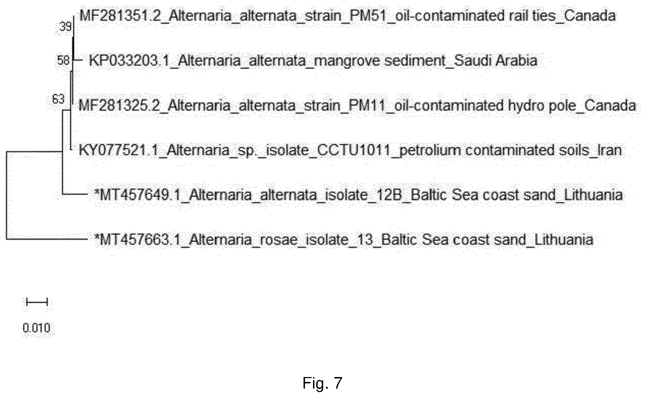

Fig. 7 shows *Alternaria* sp. strains (*) sequence similarity with sequences of fungi derived from the petroleum hydrocarbon contaminated environment or used for its degradation, from the BLAST database. Neighbour-Joining method. The percentage of replicate trees in which the associated taxa clustered together in the bootstrap test (1000 replicates) are shown next to the branches. The tree is drawn to scale, with branch lengths in the same units as those of the evolutionary distances used to infer the phylogenetic tree. The evolutionary distances were computed using the Maximum Composite Likelihood method and are in the units of the number of base substitutions per site. Codon positions included were 1st+2nd+3rd+Noncoding. All ambiguous positions were removed for each sequence pair (pairwise deletion option). Evolutionary analyses were conducted in MEGA X [4].

Preferred embodiments of the invention will be described herein below with reference to the drawings. Each figure contains the same numbering for the same or equivalent element.

### DETAILED DESCRIPTION OF THE INVENTION

### 1. Fungal strains used in the experiment

The fungi were isolated from the water and sand derived from the south-eastern Baltic Sea coast. 18 fungal isolates further were tested on water agar with addition of 250 microliters of PHs derived from oil field in Lithuania, also adding NaCl. Triplicates of each fungal isolate were inoculated under following conditions:
a) Water agar + NaCl 0.08 % + PHs + 50 microliters of fungal inoculum;
b) Water agar + PHs + 50 microliter of fungal inoculum;
c) Control (water agar + NaCl 0.08 % + PHs);
d) Control (water agar + PHs).

The Petri dishes with fungal isolates were kept in the incubator under a temperature of 20 °C for two weeks. The activity was measured at the time (day) T2, T4, T6, T8, T15. The activity of oil degradation was indicated based on two criteria: a) visual change of oil biofilm; b) the length of fungal hypha from the inoculum centre (Fig. 1 and Fig. 2).

The following fungi were indicated as potentially active toward tested oil biofilm (Table 1):

**Table 1. Fungi data which was indicated as potentially active towards petroleum degradation in experiment**

| **Fungal isolate** | **Activity towards tested petroleum film** | **Source of isolation** | **BLAST accession number** |
|---|---|---|---|
| *Emericellopsis sp.* | medium | seawater | MT457648 |
| *Penicillium sp. 1* | medium | sand | MT457709 |
| *Fusarium equiseti 1* | strong | sand | MT457652 |
| *Clonostachys rosea* | strong | sand | MT457708 |
| *Penicillium sp. 2* | medium | sand | MT457653 |
| *Fusarium equiseti 2* | strong | sand | MT457662 |
| *Alternaria alternata* | strong | sand | MT457649 |
| *Alternaria rosae* | strong | sand | MT457663 |

The DNA was extracted from the tested fungi isolates and amplified using 18S primers: ITS3 (GCATCGATGAAGAACGCAGC) and ITS4 (TCCTCCGCTTATTGATATGC). After that it was sequenced, and derived sequences compared with the sequence from the BLAST database (https://blast.ncbi.nlm.nih.gov/Blast.cgi).

Based on the literature data our tested potentially active fungal species possess different enzymes, that also could act toward PHs degradation, and the ability to produce biosurfactants (Table 2).

**Table 2. The enzymes characteristic for tested fungi species or genera based on literature data**

| **Species** | **Enzymes** |
|---|---|
| *Fusarium equiseti* | Laccase, manganese-dependent peroxidase (MnP) and manganese-independent peroxidase (MIP). |
| *Clonostachys rosea* | MnP, MIP, extracellular protease (PrC), versatile peroxidase (VP) |
| *Alternaria alternata* | Laccase, MnP, LiP and catalase enzymes. |
| *Alternaria rosea* | Laccase, MnP, LiP and catalase enzymes. |
| *Emericellopsis sp.* | Cellulase, protease, amylase, βglucosidase, lipase and laminarinase. |
| *Penicillium sp. 1* | Xynalase, cellulase, manganese dependent peroxidase (MnP), lignin peroxidase (LiP) and laccase. |
| *Penicillum sp. 2* | Xynalase, cellulase, manganese dependent peroxidase (MnP), lignin peroxidase (LiP) and laccase. |

### 2. Fungal biomass production and preservation for further use

Fungal biomass production uses wheat straw, oat straw, rice straw, corn straw, barley straw, cereal straw, wheat bran or sawdust under solid state fermentation conditions. Solid substrate fermentation technology is culturing fungi on solid waste and is used in biotechnology for production of organisms and their by-products. Sawdust substrate is suitable for production of lygnolitic enzymes of fungi such as a *Fusarium equiseti* (Nathan et al., 2018). Wheat straw is one of the most abundant crop residues in the world and successfully used for production of fungal biomass with high enzymatic activity (of peroxidases and laccases), for example of *Clonostachys rosea* or *Alternaria* sp. (Galic et al., 2018). Important feature of wheat straw is also that it can be used as a natural sorbent in aquatic oil spills (Paulauskiene and Jucike, 2015).

The inoculum of fungi is prepared by: (i) inoculation of 100 ml of synthetic medium with mycelial discs cultures from potato dextrose agar, (ii) incubation at rotary shaker (160 rpm) at room temperature (20 ± 2 °C) for 7 days, (iii) washing of obtained biomass by sterilised distilled water, and (iv) its homogenisation in laboratory blender.

For solid-state fermentation 1:5 of the solid substrate (g) should be moistened with 1:1 (ml) of water, as a nitrogen source - peptone or yeast extract could be added. Then autoclaved for 20 min at 121 °C and inoculated aseptically with 1:15 (ml) of mycelial suspension (inoculum). Incubation should be carried out at 25 ºC in x15 bigger than used biomaterial volume flasks for up to 4-7.

After that wheat straw overgrown with mycelium and conidia of fungal isolate should be preserved by lyophilization. The biomass is subjected to a freeze-drying process, preceded by external freezing to a temperature of -80 °C. After that the freeze-drying is performed with the lyophilizer, at an external manifold pressure of 0.2 mbar for 20 hours.

After preservation, lyophilizates should be transferred into plastic bags, vacuum sealed, and stored at room temperature until the deployment.

### 3. Fungal preparation use in marine environment

The fungal species were isolated from the water and sand derived from the south-eastern Baltic Sea coast; thus, this suggests that the fungi are a part of a natural ecosystem in this region. Therefore, the potential use of these particular species in bioremediation of petroleum hydrocarbons cause no harmful side effects to the environment.

The purpose of using fungi is to reduce the amount of damage done by an oil spill when it is remediated from the sea surface for it not to drift into shallow water or ashore. Most damage is done by spilled oil when it reaches shallow waters or the shore, hereby contaminating sensitive habitats and harming the species that live in these near-shore or coastal habitats. However, this method could be used in very shallow waters, less than 5 or 10 metres deep, and have no impact to the seabed and to the benthic organisms that reside there. The method could be also used in bioremediation of spilled oil, that is directly above shellfish beds, over corals, seagrass and fish spawning areas as well as on the vicinity of fish cages and shallow water fisheries beds.

The prevailing sea water quality parameters is another factor that needs to be taken into consideration. The biomass could be applied on the petroleum hydrocarbons at water temperature above +15°C, pH range from 8 to 9, salinity range from 0.1 up to 8 PSU. In order to function, the inoculum must be able to reach the spilled crude oil, oil products or alternative fuels. The inoculum in the form of solid particles is applied on the petroleum hydrocarbons by scattering it as well as in a liquid form when the inoculum is sprayed from a vessel or an aircraft (fitted with the appropriate spraying or scattering equipment) on the surface of the water at the oil spills area. The inoculum needs to be applied to spilled oil in a manner that allows the inoculum to soak into the oil product.

Furthermore, according to the invention an additional application of a specified inoculum is used for restoring the natural microflora of the affected area, for example by using *Emercilopsis sp.*

### References

R.R. Kalantary, A. Mohseni-Bandpi, A. Esrafili, S. Nasseri, F.R. Ashmagh, S. Jorfi, Ja'fari, M. Effectiveness of biostimulation through nutrient content on the bioremediation of phenanthrene contaminated soil. Iranian J. Environ. Health Sci. Eng., 12 (1) (2014), p. 143
Dellagnezze B.M., Gomes M.B., de Oliveira V.M. (2018) Microbes and Petroleum Bioremediation. In: Kumar V., Kumar M., Prasad R. (eds) Microbial Action on Hydrocarbons. Springer, Singapore
Atlas, R. M. (1995). Bioremediation of petroleum pollutants. International Biodeterioration & Biodegradation, 35(1-3), 317-327.
Faten, A. M., & Abeer, A. A. E. A. (2013). Enzyme activities of the marine-derived fungus Alternaria alternata cultivated on selected agricultural wastes. Journal of Applied Biological Sciences, 7(1), 39-46.
Head, I. M., & Swannell, R. P. (1999). Bioremediation of petroleum hydrocarbon contaminants in marine habitats. Current opinion in biotechnology, 10(3), 234-239.
Zhang, Ji, and James J. Elser. "Carbon: nitrogen: phosphorus stoichiometry in fungi: a meta-analysis." Frontiers in microbiology 8 (2017): 1281.
Gutiérrez, M. H., Vera, J., Srain, B., Quiñones, R. A., Wörmer, L., Hinrichs, K. U., & Pantoja-Gutiérrez, S. (2020). Biochemical fingerprints of marine fungi: implications for trophic and biogeochemical studies. *Aquatic Microbial Ecology,* 84, 75-90.
McGenity, T.J., Folwell, B.D., McKew, B.A. and Sanni, G.O., 2012. Marine crude-oil biodegradation: a central role for interspecies interactions. Aquatic Biosystems, 8(1), p.10.
TIP 2 Fate of Marine Oil Spills. https://www.itopf.org/knowledge-resources/documents-guides/document/tip-02-fate-of-marine-oil-spills/ (accessed on 2020)
Galic, M., Cilerdzic, J., Vukojevic, J., Stajic, M., & Brceski, I. (2018). Potential of selected micromycetes for wheat straw degradation. Journal of Environmental Protection and Ecology, 19(3), 1116-1122.
Ameen, F., Moslem, M., Hadi, S., & Al-Sabri, A. E. (2016). Biodegradation of diesel fuel hydrocarbons by mangrove fungi from Red Sea Coast of Saudi Arabia. Saudi journal of biological sciences, 23(2), 211-218.
Al-Hawash, A. B., Zhang, X., & Ma, F. (2019). Removal and biodegradation of different petroleum hydrocarbons using the filamentous fungus Aspergillus sp. RFC-1. MicrobiologyOpen, 8(1), e00619.
Chulalaksananukul, S., Gadd, G. M., Sangvanich, P., Sihanonth, P., Piapukiew, J., & Vangnai, A. S. (2006). Biodegradation of benzo (a) pyrene by a newly isolated Fusarium sp. FEMS microbiology letters, 262(1), 99-106.
Al-Hawash, A. B., Alkooranee, J. T., Abbood, H. A., Zhang, J., Sun, J., Zhang, X., & Ma, F. (2018). Isolation and characterization of two crude oil-degrading fungi strains from Rumaila oil field, Iraq. Biotechnology reports, 17, 104-109.
Effendi, A. J., Kardena, E., & Helmy, Q. (2018). Biosurfactant-Enhanced Petroleum Oil Bioremediation. In Microbial Action on Hydrocarbons (pp. 143-179). Springer, Singapore.
Colwell, R. R., Walker, J. D., & Cooney, J. J. (1977). Ecological aspects of microbial degradation of petroleum in the marine environment. CRC critical reviews in microbiology, 5(4), 423-445.
Paulauskiene, T., & Jucike, I. (2015). Aquatic oil spill cleanup using natural sorbents. Environmental Science and Pollution Research, 22(19), 14874-14881.
Tyagi, M., da Fonseca, M. M. R., & de Carvalho, C. C. (2011). Bioaugmentation and biostimulation strategies to improve the effectiveness of bioremediation processes. Biodegradation, 22(2), 231-241.
Nathan VK, Kanthimathinathan SR, Rani ME, Rathinasamy G, Kannan ND (2018). Biobleaching of waste paper using lignolytic enzyme fromFusarium equisetiVKF2: amangrove isolate. Cellulose 25(7):4179-4192.

## Claims

1. A method for petroleum hydrocarbon degradation in water using fungi, **characterized in that** petroleum hydrocarbons to be degraded are brought into contact with an inoculum formed from at least the following components:
▪ fungi strains isolated from sand and water of Baltic Sea coast samples, wherein the fungi strains are selected from *Clonostachys rosea, Alternaria rosae* or *Emericellopsis maritima*
▪ an environmentally friendly support material that acts as a solid substrate for the fungi and is a natural sorbent of petroleum hydrocarbons, wherein the support material on which at least one fungi strain is immobilized, is selected from: wheat straw, oat straw, rice straw, corn straw, barley straw, cereal straw, wheat bran or sawdust,
and wherein a fungi strain Emericellopsis *sp*. is applied additionally for microflora restoration.

2. The method according to claim 1, **characterized by** immobilizing the additional nutrients nitrogen and phosphorus on the support material before lyophilization.

3. The method according to any one of claims 1 to 2, **characterized by** using the enzyme compositions obtainable from the fungal strains in addition to the fungal strains employed, wherein the enzyme compositions are immobilized with the aid of environmentally friendly support material.

4. The method according to any one of claims 1 to 3, **characterized by** bringing the inoculum into contact with the petroleum hydrocarbons to be degraded a plurality of times, with a different composition of the inoculum with regards to fungal strains employed and/or the environmentally friendly support material employed and/or the nutrients with an additional nitrogen and phosphorus employed.

5. The method according to any one of claims 1 to 4, **characterized by** applying the inoculum in the form of solid particles on the petroleum hydrocarbons by scattering it from a vessel or an aircraft on the surface of the water.

6. The method according to any one of claims 1 to 5, **characterized by** storing the fungi strains lyophilized until deployment.

7. The method according to any one of claims 1 to 6, **characterized by** applying the inoculum on the petroleum hydrocarbons at water temperature above +15°C.

## Patentansprüche

1. Verfahren zum Abbau von Erdölkohlenwasserstoffen in Wasser unter Verwendung von Pilzen, **dadurch gekennzeichnet, dass** die abzubauenden Erdölkohlenwasserstoffe mit einem Inokulum in Kontakt gebracht werden, das aus mindestens den folgenden Bestandteilen gebildet wird:
• Pilzstämme, die aus Proben von Sand und Wasser der Ostseeküsten isoliert werden, wobei die Pilzstämme aus *Clonostachys rosea, Alternaria rosae* oder *Emericellopsis maritima* ausgewählt sind,
• ein umweltfreundliches Trägermaterial, das als ein festes Substrat für die Pilze wirkt und ein natürliches Sorptionsmittel für Erdölkohlenwasserstoffe ist, wobei das Trägermaterial, auf dem mindestens ein Pilzstamm immobilisiert ist, aus Folgenden ausgewählt ist: Weizenstroh, Haferstroh, Reisstroh, Maisstroh, Gerstenstroh, Getreidestroh, Weizenkleie oder Sägemehl,
und wobei ein Pilzstamm *Emericellopsis sp.* zusätzlich zur Wiederherstellung der Mikroflora angewendet wird.

2. Verfahren nach Anspruch 1, **gekennzeichnet durch** Immobilisieren der zusätzlichen Nährstoffe Stickstoff und Phosphor auf dem Trägermaterial vor einer Lyophilisierung.

3. Verfahren nach einem der Ansprüche 1 bis 2, **gekennzeichnet durch** Verwenden der Enzymzusammensetzungen, die zusätzlich zu den eingesetzten Pilzstämmen von den Pilzstämmen erhältlich sind, wobei die Enzymzusammensetzungen mithilfe des umweltfreundlichen Trägermaterials immobilisiert werden.

4. Verfahren nach einem der Ansprüche 1 bis 3, **gekennzeichnet durch** Inkontaktbringen des Inokulums mit den abzubauenden Erdölkohlenwasserstoffen für eine Vielzahl von Malen, mit einer anderen Zusammensetzung des Inokulums in Bezug auf die eingesetzten Pilzstämme und/oder das eingesetzte umweltfreundliche Trägermaterial und/oder die Nährstoffe mit einem zusätzlichen eingesetzten Stickstoff und Phosphor.

5. Verfahren nach einem der Ansprüche 1 bis 4, **gekennzeichnet durch** Aufbringen des Inokulums in der Form von Feststoffpartikeln auf die Erdölkohlenwasserstoffe durch Streuen davon aus einem Behälter oder einem Flugzeug auf der Wasseroberfläche.

6. Verfahren nach einem der Ansprüche 1 bis 5, **gekennzeichnet durch** Lagern der lyophilisierten Pilzstämme bis zum Einsatz.

7. Verfahren nach einem der Ansprüche 1 bis 6, **gekennzeichnet durch** Aufbringen des Inokulums auf die Erdölkohlenwasserstoffe bei einer Wassertemperatur über +15 °C.

## Revendications

1. Procédé de dégradation d'hydrocarbures de pétrole dans l'eau utilisant des champignons, **caractérisé en ce que** les hydrocarbures de pétrole à dégrader sont mis en contact avec un inoculum formé d'au moins les composants suivants :
▪ des souches de champignons isolées à partir de sable et d'eau d'échantillons de la côte de la mer Baltique, dans lequel les souches de champignons sont sélectionnées parmi *Clonostachys rosea, Alternaria rosae* ou *Emericellopsis maritima.*
▪ un matériau de support respectueux de l'environnement qui agit comme un substrat solide pour les champignons et est un sorbant naturel des hydrocarbures de pétrole, dans lequel le matériau de support sur lequel au moins une souche de champignon est immobilisée est choisi parmi : la paille de blé, la paille d'avoine, la paille de riz, la paille de maïs, la paille d'orge, la paille de céréales, le son de blé ou la sciure de bois,
et dans lequel une souche de champignon Emecellopsis *sp*. est appliquée en plus pour la restauration de la microflore.

2. Procédé selon la revendication 1, **caractérisé par** l'immobilisation des nutriments supplémentaires azote et phosphore sur le matériau de support avant lyophilisation.

3. Procédé selon l'une des revendications 1 à 2, **caractérisé par** l'utilisation des compositions enzymatiques pouvant être obtenues à partir des souches fongiques en plus des souches fongiques utilisées, dans lequel les compositions enzymatiques sont immobilisées à l'aide d'un matériau de support respectueux de l'environnement.

4. Procédé selon l'une quelconque des revendications 1 à 3, **caractérisé par** la mise en contact de l'inoculum avec les hydrocarbures de pétrole à dégrader une pluralité de fois, avec une composition de l'inoculum différente en ce qui concerne les souches fongiques employées et/ou le matériau de support respectueux de l'environnement utilisé et/ou les nutriments avec un azote et un phosphore supplémentaires utilisés.

5. Procédé selon l'une quelconque des revendications 1 à 4, **caractérisé par** l'application de l'inoculum sous forme de particules solides sur les hydrocarbures de pétrole en le diffusant depuis un navire ou un aéronef à la surface de l'eau.

6. Procédé selon l'une quelconque des revendications 1 à 5, **caractérisé par** le stockage des souches de champignons lyophilisées jusqu'à leur déploiement.

7. Procédé selon l'une quelconque des revendications 1 à 6, **caractérisé par** l'application de l'inoculum sur les hydrocarbures de pétrole à une température de l'eau supérieure à +15 °C.
